# EUROPEAN PATENT SPECIFICATION

(11) **EP 0 713 702 B1**
(45) Date of publication and mention of the grant of the patent: **29.09.2004**
(21) Application number: 95913355.4
(22) Date of filing: 28.03.1995
(51) Int. Cl.: A61K 31/40, A61K 31/47, A61K 31/435, A61K 31/55, A61P 25/22

(54) **ANXIOLYTIC AGENT**
ANXIOLYTISCHER WIRKSTOFF
AGENT ANXIOLYTIQUE

(30) Priority: 28.03.1994 JP 8393494
(43) Date of publication of application: 29.05.1996
(73) Proprietor: TAIHO PHARMACEUTICAL COMPANY LIMITED, Chiyoda-ku, Tokyo 101-8444 (JP)
(72) Inventor: YAMAMOTO, Junji, Tokushima-shi, Tokushima-ken 770 (JP); ARIMA, Takashi, Sapporo-shi, Hokkaido 062 (JP); KASAHARA, Nobuo, Tokushima-shi, Tokushima-ken 771-01 (JP); NANRI, Masato, Tokushima-shi, Tokushima-ken 771-01 (JP)
(74) Representative: Barz, Peter, Dr.
(86) International application number: PCT/JP1995/000575
(87) International publication number: WO 1995/026187

(56) References cited:
- WO-A-91/11434
- J. NEUROPSYCHIATRY CLIN. NEUROSCI., 1989, 1/3 (253-262), USA, XP000613018 PEROUTKA S.J. ET AL: "The clinical utility of pharmacological agents that act at serotonin receptors"

## Description

### TECHNICAL FIELD

The present invention relates to anxiolytic agents comprising a bicyclolactam derivative as their effective component and to the treatment of anxiety.

### BACKGROUND ART

With rapid diversification of the social environment in recent years, an increasing number of people are suffering from anxiety, and it has been expected to develop psychosomatic therapies and excellent therapeutic agents.

Benzodiazepine compounds such as diazepam have found wide use as anxiolytics. This group of agents, however, generally have side effects such as hypnotic effect, muscle relaxant effect and sedative effect. Serotonin anxiolytic agents such as buspirone are also recently developed as anxiolytics which are different from the benzodiazepine compounds in activity mechanism. Reportedly these serotonin agents are generally lesser than the benzodiazepines in side effects such as hypnotic, muscle relaxant and sedative effects, but they are lower in anxiolytic effect and have the problems of diminishing voluntary movements presumably owing to their activity as a dopamine antagonist, and causing serotonin syndrome which appears attributable to their properties as a full agonist for serotonin 1A receptor.

On the other hand, the bicyclolactam derivatives of the present invention are known compounds disclosed in International Public Disclosure No. WO 91/11434, and are known to have a cerebral function improving effect, cerebral metabolism activating or anoxic brain damage protecting effect and effect against senile dementia. Nevertheless, nothing whatsoever is known about the anxiolytic effect of these compounds.

Anxiety is an essential symptom of neuroses and is a neurotic disorder involving no organic disorder of the brain.

In contrast, the term dementia refers to an organic mental disorder which is the sustained deterioration of acquired intelligence due to an organic disorder of the brain. Many of dementias are caused by a wide variety of organic disorders of the brain and involve general deterioration of mental functions such as memory, calculation, orientation and discretion, leading to failure of correct recognition, judgment or behavior as to one's own situation.

"Diagnostic and Statistical Manual of Mental Disorders," Revised 3rd Ed. (DSM-III -R), widely used as a diagnostic manual and published by American Psychiatric Association in 1987, clearly classifies these diseases in the chapters "Anxiety Disorders (or Anxiety and Phobic Neuroses)" and "Organic Mental Syndromes and Organic Mental Disorders" (see "DSM-III -R Classification of Mental Disorders and Diagnostic Manual," (2nd Ed.), translated by Saburo Takahasi et al., published by Igaku Shoin (1988), pp. 71∼ 94 and 121∼ 129).

An object of the present invention is to provide a novel anxiolytic agent comprising a bicyclolactam derivative as its effective component.

We have investigated the pharmacological activities of bicyclolactam derivatives under different aspects and found that these compounds have a very high anxiolytic effect and are greatly diminished in side effects such as hypnotic, muscle relaxant and sedative effects.

Accordingly, the present invention relates to the use of a bicyclolactam derivative represented by the following formula 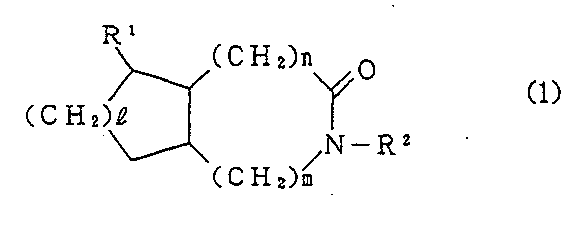 wherein R¹ is a hydrogen atom or hydroxyl group, R² is a benzoyl group which may optionally have at least one substituent, l is 1 or 2, m is 0 or 1 and n is 0, 1 or 2, provided that m and n do not represent 0 simultaneously, in the manufacture of a medicament for treating anxiety.

The present invention also relates to the novel bicyclolactam derivatives of formula (1) wherein R¹ is a hydroxyl group and R², l, m and n are as defined above, as well as to pharmaceutical compositions comprising these novel bicyclolactam derivatives and a conventional pharmaceutical carrier or excipient.

Existing as bicyclolactam derivatives of the formula (1) are stereoisomers due to the presence of the bicyclo ring and also geometric isomers and optical isomers due to the presence of the carbon atom at the bridgehead position of the bicyclo ring and the carbon atom having R¹ attached thereto. The present invention includes all of these isomers.

In view of the numbers l, m and n, the following ten kinds of bicyclo ring skeletons can be present in the compounds of the formula (1). The invention includes all of these cases.

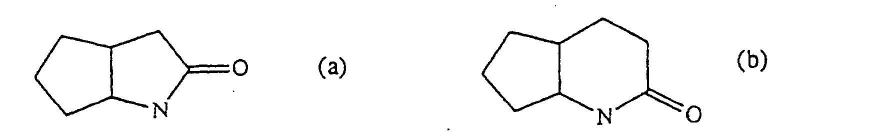

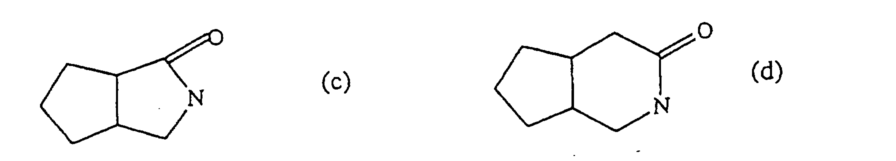

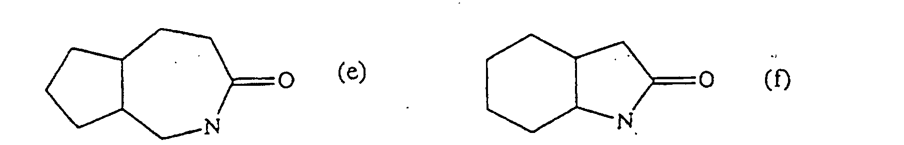

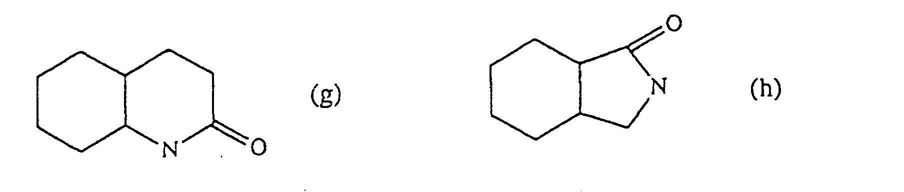

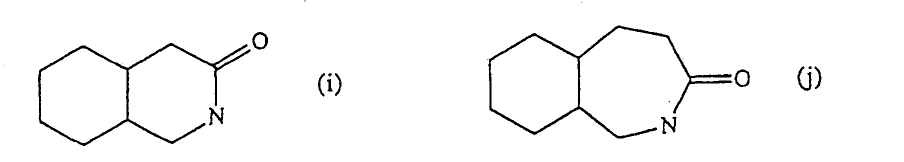

Preferable among these is the case wherein the skeleton is (a), (b), (f), (g) or (h). More preferable is the case wherein m is 0, i.e., (a), (b), (f) or (g). The most preferable is (b), (f) or (g).

According to the invention, examples of substituents which may be present in the benzoyl group represented by R² are a halogen atom, lower alkyl group, lower alkoxyl group, nitro group, cyano group, hydroxyl group or amino group. Examples of halogen atoms are fluorine, chlorine, bromine and iodine atom, among which chlorine atom is preferable. Examples of useful lower alkyl groups are straight-chain or branched alkyl groups having 1 to 6 carbon atoms, such as methyl, ethyl, n-propyl, isopropyl, n-butyl, isobutyl, tert-butyl, pentyl, isopentyl and hexyl group. Preferable among these are methyl and ethyl groups. Methyl group is more preferable. Examples of useful lower alkoxyl groups are straight-chain or branched alkoxyl groups having 1 to 6 carbon atoms, such as methoxy, ethoxy, n-propoxy, isopropoxy, n-butoxy, isobutoxy, sec-butoxy, tert-butoxy, pentyloxy, isopentyloxy and hexyloxy group, among which methoxy and ethoxy groups are preferable. Methoxy group is more preferable.

Preferable as R² of the compound represented by the formula (1) is a benzoyl group having as a substituent a halogen atom, lower alkyl group, lower alkoxyl group, nitro group, cyano group or amino group, more preferably a benzoyl group having lower alkoxyl group. Especially preferable is a benzoyl group having methoxy group. The number of substituents is preferably 1 to 3. The substituent may be present at any of the ortho-, meta- and para-positions on the phenyl ring of the benzoyl group.

Among the compounds of the formula (1), preferable are those wherein m is 0, and more preferable are those wherein R² is a benzoyl having lower alkoxyl group and wherein l is 1, m is 0 and n is 2, or l is 2, m is 0 and n is 1, or l is 2, m is 0 and n is 2. More preferable are those wherein R² is a benzoyl group having methoxy group and wherein l is 1, m is 0 and n is 2, or l is 2, m is 0 and n is 1, or l is 2, m is 0 and n is 2. In the case where R¹ is hydroxyl group, especially preferred compounds are those wherein l is 1, m is 0 and n is 2.

Examples of the compound of the above formula (1) are
2-(4-methoxybenzoyl)-2-azabicyclo[3.3.0]octane-3-one,
2-(4-methoxybenzoyl)-2-azabicyclo[4.3.0]nonane-3-one,
2-(p-toluyl)-2-azabicyclo[4.3.0]nonane-3-one,
2-(3,4-dichlorobenzoyl)-2-azabicyclo[4.3.0]nonane-3-one,
2-(3,5-dimethoxybenzoyl)-2-azabicyclo[4.3.0]nonane-3-one,
2-(4-cyanobenzoyl)-2-azabicyclo[4.3.0]nonane-3-one,
2-(4-nitrobenzoyl)-2-azabicyclo[4.3.0]nonane-3-one,
2-(4-aminobenzoyl)-2-azabicyclo[4.3.0]nonane-3-one,
2-(4-chlorobenzoyl)-2-azabicyclo[4.3.0]nonane-3-one,
8-(4-methoxybenzoyl)-8-azabicyclo[4.3.0]nonane-7-one,
2-(4-hydroxybenzoyl)-2-azabicyclo[4.3.0]nonane-3-one,
3-(4-methoxybenzoyl)-3-azabicyclo[3.3.0]octane-4-one,
3-(4-methoxybenzoyl)-3-azabicyclo[4.3.0]nonane-4-one,
3-(4-methoxybenzoyl)-3-azabicyclo[5.3.0]decane-4-one,
3-(4-methoxybenzoyl)-3-azabicyclo[4.4.0]decane-4-one,
3-(4-methoxybenzoyl)-3-azabicyclo[5.4.0]undecane-4-one,
7-(4-methoxybenzoyl)-7-azabicyclo[4.3.0]nonane-8-one,
7-(p-toluyl)-7-azabicyclo[4.3.0]nonane-8-one,
7-(3,4-dichlorobenzoyl)-7-azabicyclo[4.3.0]nonane-8-one,
7-(3,5-dimethoxybenzoyl)-7-azabicyclo[4.3.0]nonane-8-one,
7-(4-cyanobenzoyl)-7-azabicyclo[4.3.0]nonane-8-one,
7-(4-nitrobenzoyl)-7-azabicyclo[4.3.0]nonane-8-one,
7-(4-aminobenzoyl)-7-azabicyclo[4.3.0]nonane-8-one,
7-(4-chlorobenzoyl)-7-azabicyclo[4.3.0]nonane-8-one,
7-(4-hydroxybenzoyl)-7-azabicyclo[4.3.0]nonane-8-one,
2-(4-methoxybenzoyl)-2-azabicyclo[4.4.0]decane-3-one,
2-(p-toluyl)-2-azabicyclo[4.4.0]decane-3-one,
2-(3,4-dichlorobenzoyl)-2-azabicyclo[4.4.0]decane-3-one,
2-(3,5-dimethoxybenzoyl)-2-azabicyclo[4.4.0]decane-3-one,
2-(4-cyanobenzoyl)-2-azabicyclo[4.4.0]decane-3-one,
2-(4-nitrobenzoyl)-2-azabicyclo[4.4.0]decane-3-one,
2-(4-aminobenzoyl)-2-azabicyclo[4.4.0]decane-3-one,
2-(4-chlorobenzoyl)-2-azabicyclo[4.4.0]decane-3-one,
2-(4-hydroxybenzoyl)-2-azabicyclo[4.4.0]decane-3-one,
7-hydroxy-2-(4-methoxybenzoyl)-2-azabicyclo[4.3.0]nonane-3-one,
2-hydroxy-7-(4-methoxybenzoyl)-7-azabicyclo[4.3.0]nonane-8-one and
7-hydroxy-2-(4-methoxybenzoyl)-2-azabicyclo[4.4.0]decane-3-one.

Preferable examples are
2-(4-methoxybenzoyl)-2-azabicyclo[4.4.0]decane-3-one,
2-(4-methoxybenzoyl)-2-azabicyclo[3.3.0]octane-3-one,
2-(4-methoxybenzoyl)-2-azabicyclo[4.3.0]nonane-3-one,
2-(p-toluyl)-2-azabicyclo[4.3.0]nonane-3-one,
2-(3,4-dichlorobenzoyl)-2-azabicyclo[4.3.0]nonane-3-one,
2-(3,5-dimethoxybenzoyl)-2-azabicyclo[4.3.0]nonane-3-one,
2-(4-cianobenzoyl)-2-azabicyclo[4.3.0]nonane-3-one,
2-(4-nitrobenzoyl)-2-azabicyclo[4.3.0]nonane-3-one,
2-(4-aminobenzoyl)-2-azabicyclo[4.3.0]nonane-3-one,
2-(4-chlorobenzoyl)-2-azabicyclo[4.3.0]nonane-3-one,
7-(4-methoxybenzoyl)-7-azabicyclo[4.3.0]nonane-8-one,
8-(4-methoxybenzoyl)-8-azabicyclo[4.3.0]nonane-7-one and
7-hydroxy-2-(4-methoxybenzoyl)-2-azabicyclo[4.3.0]nonane-3-one,

More preferable examples are
2-(4-methoxybenzoyl)-2-azabicyclo[4.4.0]decane-3-one,
2-(4-methoxybenzoyl)-2-azabicyclo[4.3.0]nonane-3-one,
7-(4-methoxybenzoyl)-7-azabicyclo[4.3.0]nonane-8-one and
7-hydroxy-2-(4-methoxybenzoyl)-2-azabicyclo[4.3.0]nonane-3-one.

The bicyclolactam derivatives of the formula (1) wherein R⁴ is a hydrogen atoms are known compounds which are disclosed for example in WO 91/11434 (US-A-5185344, US-A-5214039). etc.

The bicyclolactam derivative (1-a) of the present invention wherein R¹ is hydrogen atom can be prepared, for example, by the following reaction process 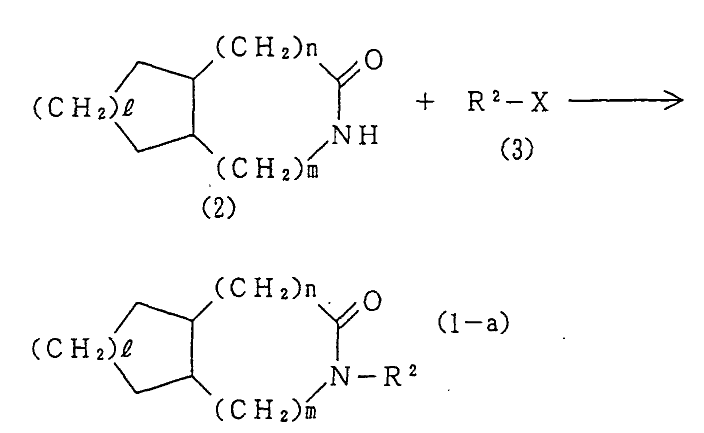 wherein R², ℓ, m and n are as defined above, X is halogen atom.

Bicyclolactam compound (2) is a known compound and is easily prepared by methods disclosed in Journal of American Chemical Society, 77, 409 (1955), Yakugaku Zasshi, 84, 674 (1964) and Journal of Chemical Society Perkin Transactions I 11, 2563 (1982). The compound of the formula (1-a) can be prepared by reacting the bicyclolactam compound (2) with the halide compound (3), in the presence of a base in an appropriate solvent.

The solvent is not limited specifically insofar as it does not participate in the reaction. Examples of solvents generally useful are hydrocarbon halides such as dichlorolmethane and chloroform, ethers such as ethyl ether and tetrahydrofuran, aromatic hydrocarbons such as benzene and toluene, aprotic polar solvents such as N,N-dimethylformamide and dimethylsulfoxide.

As to the proportion of the compound (2) and the halide compound (3), it is usual to use 0.5 to 2 moles, preferably one mole of the compound (3) per mole of the compound (2). Examples of bases are organic amines such as triethylamine, pyridine and 4-dimethylaminopyridine, and inorganic bases such as sodium hydride and sodium amide. The amount of the base is usually 0.5 to 2 moles, preferably one mole per mole of the compound (2). The reaction temperature is 0 to 150 °C , preferably 50 to 100 °C . The reaction time is 1 to 48 hours, preferably 2 to 12 hours.

The bicyclolactam derivative (1-b) of the present invention wherein R¹ is hydroxyl group can be prepared, for example, by the following reaction process 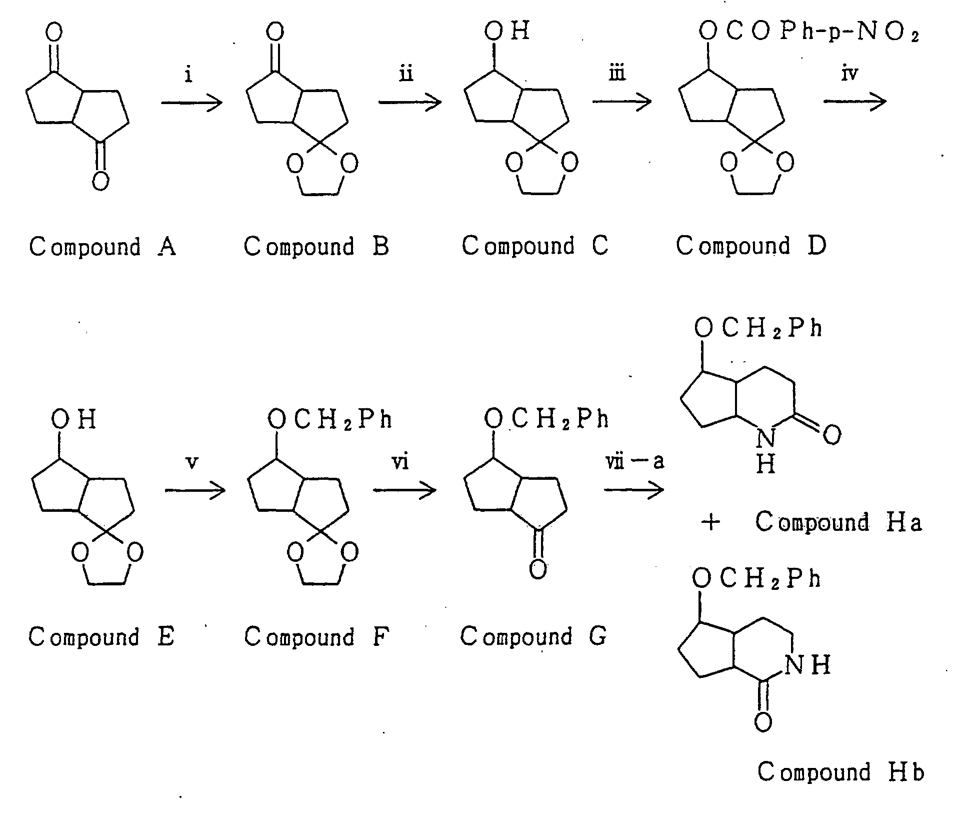 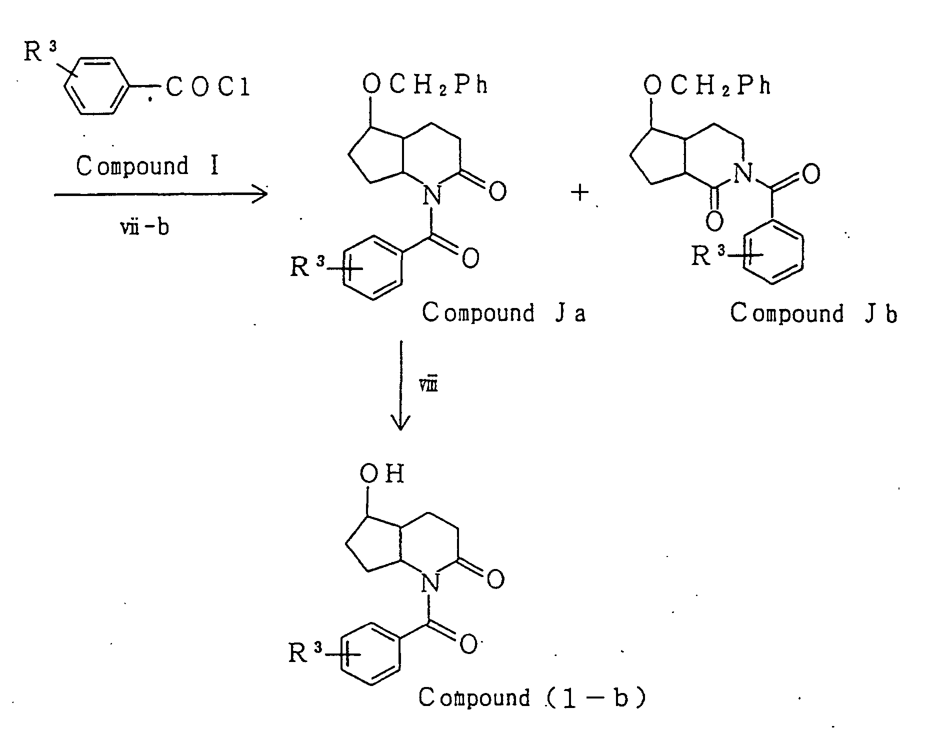 wherein R³ is hydrogen atom, halogen atom, lower alkyl group, lower alkoxyl group, nitro group, cyano group, hydroxyl group or amino group.
(i) A known compound A disclosed in J. Org. Chem., 42, 3764∼ 3767(1977) is reacted with ethylene glycol in a suitable solvent in the presence of an acid catalyst to obtain a compound B. The solvent to be used is not particularly limited insofar as it does not participate in the reaction; it is, for example, an aromatic hydrocarbon such as benzene, toluene or xylene. Examples of useful acid catalysts are sulfuric acid, p-toluenesulfonic acid, methanesulfonic acid, trifluoromethanesulfonic acid the like. The reaction is conducted using ethylene glycol and the acid catalyst each in an amount of about 1 to about 2 moles per mole of the compound A. The reaction temperature is 80 °C to a temperature around the boiling point of the solvent. For the completion of the reaction, the reaction time is 1 to 8 hours, preferable about 4 to about 7 hours. The compound B obtained by the invention can be used for the subsequent reaction, as isolated or without being isolated.
(ii) Next, the compound B is reacted with a reducing agent in a suitable solvent to obtain a compound C. The solvent to be used is not limited specifically insofar as it does not participate in the reaction. Examples of such solvents are methanol, ethanol, propanol, isopropanol and like alcohols, dioxane, 1,2-dimethoxyethane, tetrahydrofuran and like ethers. Examples of useful reducing agents are lithium aluminum hydride, diisobutyl aluminum hydride, diborane, sodium boron hydride and the like. The reaction is conducted using the reducing agent in about 1 to about 1.5 moles per mole of the compound C. The reaction temperature is -5 °C to room temperature, preferably about 0 to about 10 °C. The reaction time is preferably about 1 to about 3 hours. The compound C resulting from the reaction can be used for the subsequent reaction, as isolated or without being isolated.
(iii) The compound C is reacted with p-nitrobenzoic acid, triphenylphosphine and diethyl azodicarboxylate in a suitable solvent to obtain a compound D. The solvent to be used is not limited specifically insofar as it does not participate in the reaction. Examples of such solvents are dioxane, 1,2-dimethoxyethane, tetrahydrofuran and like ethers, chloroform, dichloromethane, dichloroethane and like hydrocarbon halides. The reaction is conducted using the latter three reactants each in about 1 to about 3 moles per mole of the compound C. The reaction temperature is -5 to 50 °C, preferably about 0 °C to around room temperature. The reaction times is 1 to 15 hours, preferably about 6 to about 12 hours. The compound D resulting from the reaction can be used for the subsequent reaction, as isolated or without being isolated.
(iv) The compound D is hydrolyzed in a suitable solvent with use of an anion exchange resin to obtain a compound E. The solvent to be used is not limited specifically insofar as it will not participate in the reaction. Examples of such solvents are methanol, ethanol, propanol, isopropanol and like alcohols. The reaction is conducted using the anion exchange resin in about 1 to about 10 moles per mole of the compound D. The reaction temperature is room temperature to 100 °C, and the reaction time is about 10 to about 24 hours. The compound E resulting from the reaction can be used for the subsequent reaction, as isolated or without being isolated.
(v) The compound E is reacted with benzyl bromide in a suitable solvent in the presence of a base to obtain a compopund F. The solvent to be used is not limited specifically insofar as it does not participate in the reaction. Examples of such solvents are N,N-dimethylformamide, N,N-dimethylacetamide, acetonitrile and like aprotic polar solvents, dioxane, 1,2-dimethoxyethane, tetrahydrofuran and like ethers. Examples of useful bases are trimethylamine, triethylamine, pyridine and like tertiary amines, potassium carbonate, sodium carbonate and like alkali metal carbonate, and potassium hydride, sodium hydride and like alkali metal hydrides. For the reaction, the base and benzyl bromide are used each in about 1 to about 2 moles per mole of the compound E. The reaction temperature is room temperature to 100 °C, preferably room temperature to about 70 °C. The reaction time is 8 to 30 hours, preferably about 20 to about 28 hours. The compound F resulting from the reaction can be used for the subsequent reaction, as isolated or without being isolated.
(vi) The compound F is subjected to a ketal removing reaction in a suitable solvent with use of an acid to obtain a compound G. The solvent is not limited specifically insofar as it does not participate in the reaction. Examples of solvents are alcohols such as methanol, ethanol, propanol and isopropanol, and ethers such as dioxane, 1,2-dimethoxyethane and tetrahydrofuran. Examples of useful acids are acetic acid, trifluoroacetic acid, oxalic acid and like organic acids, hydrochloric acid, bromic acid, sulfuric acid, nitric acid and like inorganic acids. The reaction temperature is 0 to 60 °C, preferably about 10 to about 70 °C. The reaction time is about 2 to about 8 hours. The compound G resulting from the reaction can be used for the subsequent reaction, as isolated or without being isolated.
(vii - a) The compound G is reacted with hydroxylamine and sodium acetate in a suitable solvent to obtain an oxime of the compound G. The solvent is not limited specifically insofar as it does not participarte in the reaction. Examples of useful solvents are methanol, ethanol, propanol, isopropanol and like alcohols, dioxane, 1,2-dimethoxyethane, tetrahydrofuran and like ethers. Hydroxylamine and sodium acetate are used each in about 1.5 to 2 moles per mole of the compoudn G. The reaction temperature is 0 to 50 °C, preferably room temperature. The reaction time is preferably 5 to 8 hours.
   Subsequently, the resulting oxime of the compound G is reacted with p-toluenesulfonyl chloride in a suitable solvent in the presence of a base to obtain a p-tosylic acid ester of the compound G. Silica gel is added to the ester in the same solvent, followed by a Beckmann rearrangement reaction to obtain a mixture of compound Ha and compound Hb. The solvent to be used is not limited specifically insofar as it does not participate in the reaction. Examples of such solvents are benzene, toluene, xylene and like aromatic hydrocarbons, chloroform, dichloromethane, dichloroethane and like hydrocarbon halides. Examples of useful bases are trimethylamine, triethylamine, pyridine and like tertiary amines. For the reaction, the base and p-tosyl chloride are used each in 2 to 3 moles per mole of the oxime of the compound G. The reaction temperature for tosylation is about 0 to 10 °C, and the reaction time is about 4 to about 8 hours. The Beckmann rearrangement reaction in silica gel is conducted at a temperature of about 10 to about 30 °C for about 12 to about 24 hours.
(vii -b) The resulting mixture is reacted with a compound I in a suitable solvent in the presence of a base to obtain a compound Ja and compound Jb. The solvent is not limited specifically insofar as it does not participate in the reaction. Examples of useful solvents are chloroform, dichloromethane, dichloroethane and like hydrocarbon halides. Examples of useful bases are tertiary amines such as trimethylamine, triethylamine and pyridine. For the reaction, the base is used in about 1 to about 2 moles per mole of the mixture. The reaction temperature is about 5 to about 50 °C, preferably about 10 °C to around room temperature. The reaction time is 12 to 36 hours, preferably about 24 to about 36 hours. The compound Ja or compound Jb resulting from the reaction can be used for the subsequent reaction, as isolated or without being isolated.
(viii) The compound Ja is hydrogenated in a suitable solvent in the presence of palladium-carbon to obtain a compound (1-b). The solvent is not limited specifically insofar as it does not participate in the reaction. Examples of useful solvents are methanol, ethanol, propanol, isopropanol and like alcohols, dioxane, 1,2-dimethoxyethane, tetrahydrofuran and like ethers, and methyl acetate, ethyl acetate and like acetic acid esters. For the reaction, palladium-carbon is used preferably in the ratio of 0.5 to 1 by weight based on the compound Ja. The reaction temperature is preferably around room temperature to about 50 °C. The reaction time is about 10 to about 20 hours.

The compound (1) thus obtained can be isolated and purified by a usual method such as recrystallization or column chromatography. The racemic compound obtained can be divided into the desired optical isomers, for example, by fractional recrystallization for the separation of salts from optically active acids or by passing a column packed with an optically active carrier. The stereoisomers can be individually separated off and purified by a usual method such as fractional crystallization or chromatography.

The anxiolytic agent embodying the present invention can be given orally or parenterally to mammals including man. The pharmaceutical preparations of the present invention are not limited specifically in the unit form of administration but can be in various forms in conformity with preventive or therapeutic purposes. These forms of preparations include, for example, oral preparations, injections, suppositories, external preparations (such as poultices and like plasters, ointments, creams and lotions), eye drops, nasal drops or sprays, etc.

The anxiolytic agent of the present invention is prepared and used in the form of a composition having a desired conventional pharmaceutical carrier or excipient incorporated therein by a usual method.

Stated more specifically, examples of carriers for use in formulating the agent as tablets, encapsulated preparations, granules, powders, etc. for oral administration are excipients such as lactose, sucrose, sodium chloride, glucose, urea, starch, calcium carbonate, kaolin, crystalline cellulose and silicic acid, binders such as water, ethanol, propanol, syrup, glucose solution, starch solution, gelatin solution, carboxymethyl cellulose, hydroxypropyl cellulose, hydroxypropyl starch, shellac, methyl cellulose, ethyl cellulose, potassium phosphate and polyvinylpyrrolidone, disintegrators such as dried starch, sodium alginate, agar powder, laminaria powder, sodium hydrogencarbonate, calcium carbonate, polyoxyethylene sorbitan fatty acid esters, sodium laurylsulfate, stearic acid monoglyceride, starch and lactose, disintegration suppressants such as sucrose, stearic acid, cacao butter and hydrogenated oils, absorption promotors such as quaternary ammonium bases and sodium laurylsulfate, humectants such as glycerin and starch, absorbents such as starch, lactose, kaolin, bentonite and colloidal silicic acid, glazing agents such as purified talc, stearic acid salts, boric acid powder and polyethylene glycol, corrigents such as sucrose, bitter orange peel, citric acid and tartaric acid, etc. When required, the tablets can be those having a usual coating, such as sugar-coated tablets, gelatin-coated tablets, enteric-coated tablets, film-coated tablets, double-layer tablets and multi-layer tablets. The encapsulated preparation is made by mixing the present compound with carriers such as those exemplified above and filling the mixture into hard gelatin capsules or soft capsules.

Liquid preparations for oral administration include aqueous or oily suspensions, solutions, syrups and elixirs, and are prepared in the usual manner by adding a corrigent, buffer, stabilizer, flavoring agent, to the present compound. In this case, examples of useful corrigents are those exemplified above, useful buffers include sodium citrate, and useful stabilizers include tragacanth, gum arabic and gelatin, etc.

Injections are aqueous or oily suspensions and solutions, or powdery fillers and freeze-dried preparations which are dissolved when to be used. Injections are prepared in the usual manner by adding to the present compound a pH adjusting agent, buffer, stabilizer, isotonic agent, diluent, local anesthetic, etc. Examples of pH adjusting agents and buffers for use in this case are sodium citrate, sodium acetate, sodium phosphate and the like. Examples of useful stabilizers are sodium pyrosulfite, EDTA, thioglycolic acid, thiolactic acid, etc. Examples of useful diluents are water, aqueous solution of lactic acid, ethyl alcohol, propylene glycol, ethoxylated isostearyl alcohol, polyoxyisostearyl alcohol, polyoxyethylene sorbitan fatty acid ester, etc. Examples of useful local anesthetics are procaine hydrochloride, lidocaine hydrochloride, etc.

In preparing suppositories, use can be made of carriers such as polyethylene glycol, lanolin, cacao fat, esters of higher alcohols, gelatin, semisynthetic glyceride, etc., and when required, surfactants such as Tween (trademark).

Ointments (pastes, creams, gels, etc.) are prepared by admixing with the present compound a base, stabilizer, lubricant, preservative, etc. which are usually used. Examples of bases are fluid paraffin, white petrolatam, bleached beeswax, octyldodecyl alcohol, paraffin and the like. Examples of useful preservatives are methyl p-hydroxybenzoate, ethyl p-hydroxybenzoate, propyl p-hydroxybenzoate and the like.

Plasters are prepared by applying the ointment, cream, gel, paste or the like to a usual support in the conventional manner. Examples of suitable supports are woven or nonwoven fabrics of cotton, staple fiber or chemical fiber, films of flexible polyvinyl chloride, polyethylene, polyurethane or the like, and foamed sheets of such material.

When required, the foregoing preparations may have further incorporated therein a coloring agent, preservative, perfume, flavoring, sweetener and the like, and other medicinals.

The method of administering the pharmaceutical preparation of the invention is not limited specifically but determined according to the form of preparation, age, sex and other conditions of the patient and degree of symptom of the patient. For example, tablets, pellets, powders, solutions, suspensions, emulsions, granules and capsules are given orally. Suppositories are introduced into the rectum. Injections are intravenously given singly or as mixed with a usual auxiliary solution such as glucose or amino acid solution. Further when required, they are singly administered intra-arterially, intramuscularly, intracutaneously, subcutaneously or intraperitoneally. Ointments are applied to the skin, mucous membrane of the oral cavity, etc. Plasters are applied to the skin.

The dosage of the effective component of the preparation of the invention can be suitably determined according to the mode of administration, age, sex and other conditions of the patient and degree of the symptom. Generally the effective component is administered at a daily dose usually of 0.001 to 10 mg/kg body weight, preferably 0.01 to 5 mg/kg body weight. The present preparation can be given once or in about 2 to about four divided doses per day.

The present invention will be described below with reference to reference examples, examples and test examples. However, the invention is not limited by these examples.

### Reference Example 1

In 100 ml of dichloromethane were dissolved 3.0 g (19.6 mmol) of 2-azabicyclo[4.4.0]decane-3-one [Journal of American Chemical Society, 77, 409 (1955)], 3.35 g (19.6 mmol) of p-methoxybenzoyl chloride and 2.38 g (23.5 mmol) of triethylamine and the solution was heated under reflux for 2 hours. After cooled, an organic layer was washed with water, 10 % hydrochloric acid and saturated aqueous solution of sodium chloride, and dried over anhydrous magnesium sulfate. After removing the solvent, the resulting residue was chromatographed over silica gel to obtain 4.5 g (yield 80 %) of 2-(4-methoxybenzoyl)-2-azabicyclo[4.4.0]decane-3-one (Compound 1) from hexane-ethyl acetate (3:1) eluate. Table 1 shows melting point and yield of the compound.

### Reference Example 2

Compound 2 was obtained in the same manner as in Reference Example 1 with use of, as a starting material, 2-azabicyclo[3.3.0]octane-3-one [Yakugaku Zasshi, 84, 674 (1964)]. Table 1 shows melting point and yield of the compound.

### Reference Example 3

A known compound, 2-azabicyclo[4.3.0]nonane-3-one was prepared by the method disclosed in Journal of American Chemical Society, 77, 409 (1955).

Namely, to a solution of 50 ml (0.35 mol) of ethyl cyclopentanone-2-carboxylate in 130 ml of dioxane was added 3.8 ml of Triton B. Then, to the solution was added 27.1 ml (0.242 mol) of acrylonitrile in 50 ml of dioxane. The solution was stirred at room temperature for 12 hours, and extracted with ether after 100 ml of 10 % hydrochloric acid was added thereto. An organic layer was dried over anhydrous magnesium silfate. After removing the solvent, 300 ml of conc. hydrochloric acid was added to the residue and the mixture was heated under reflux for 24 hours. After cooling, the mixture was extracted with ether and 5 % aqueous solution of sodium hydroxide was added to the ether layer and the mixture was stirred. An aqueous layer was made acidic with addition of 10 % hydrochloric acid, and then the mixture was extracted with ethyl acetate. An organic layer was dried over anhydrous magnesium sulfate. After removing the solvent, the residue was dissolved in 150 ml of ethanol and 10 ml of conc. sulfuric acid was added thereto. The mixture was heated under reflux for 14 hours, and then ethanol was removed therefrom after cooling, and ethyl acetate was added thereto. The mixture was washed with 10 % aqueous solution of sodium hydroxide and dried over anhydrous magnesium sulfate. After removing the solvent, the resulting residue was chromatographed over silica gel to obtain 45 g (yield 70 %) of ethyl 2-oxocyclopentanepropionate from hexane-ethyl acetate (4:1) eluate. In 150 ml of 80 % ethanol was dissolved 5.5 g (30 mmol) of this compound. To the solution were added 4.17 g (60 mmol) of hydroxylamine hydrochloride and 2.7 g (33 mmol) of sodium acetate and the mixture was stirred at room temperature over night. After removing ethanol, the mixture was extracted with ethyl acetate and dried over anhydrous magnesium sulfate. After removing the solvent, the resulting residue was chromatographed over silica gel to obtain 5 g (yield 84 %) of ethyl 2-hydroxyiminocyclopentanepropionate from hexane-ethyl acetate (6:1) eluate. In 15 ml of anhydrous ethanol was dissolved 4.4 g of this compound and the solution was stirred at 50 °C for 4 hours under a hydrogen pressure of 120 atm. with use of Raney nickel (W2) as a catalyst. After removing Raney nickel by filtration and removing the solvent, the resulting residue was chromatographed over silica gel to obtain 0.95 g (yield 31 %) of 2-azabicyclo[4.3.0]nonane-3-one from ethyl acetate eluate.

Compounds 3 to 10 were obtained in the same manner as in Reference Example 1 with use of the above compound as a starting material.

### Reference Example 4

A known compound, 7-azabicyclo[4.3.0]nonane-8-one was prepared by the method disclosed in Yakugaku Zasshi, 84, 674 (1964).

Namely, the desired 7-azabicyclo[4.3.0]nonane-8-one was prepared in the same manner as in Reference Example 3, with use of, as a starting material, ethyl 2-oxocyclohexyl acetate.

Compound 11 was obtained in the same manner as in Reference Example 1 with use of the above compound as a starting material.

### Reference Exmaple 5

Compound 12 was obtained in the same manner as in Reference Example 1 with use of, as a starting material, 8-azabicyclo[4.3.0]nonane-7-one [Journal of Chemical Society Perkin Transactions I 11, 2563 (1982)). Table 1 shows melting point and yield of the compound.

In Table 1, Me and OMe stand for methyl and methoxy respectively.

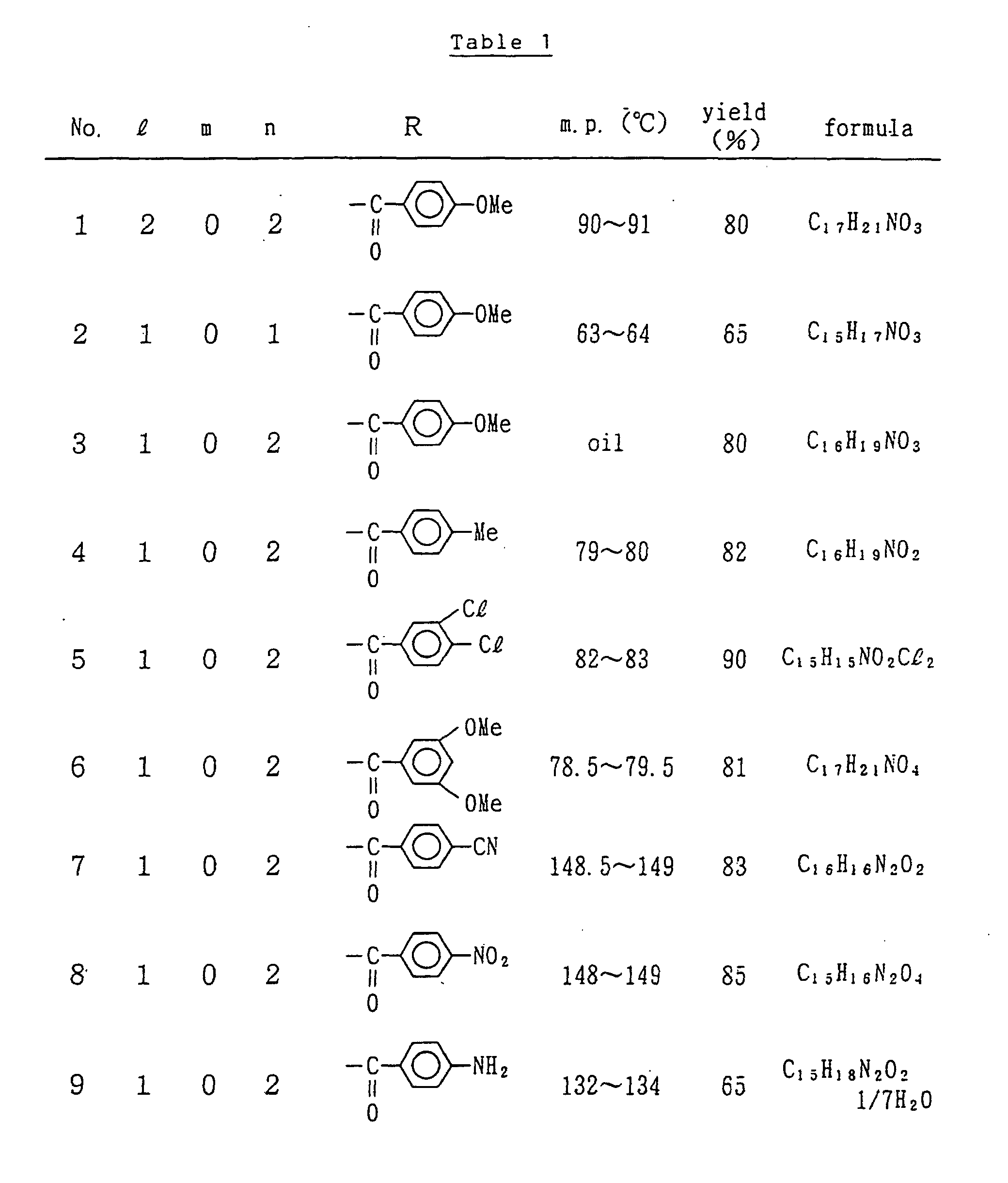

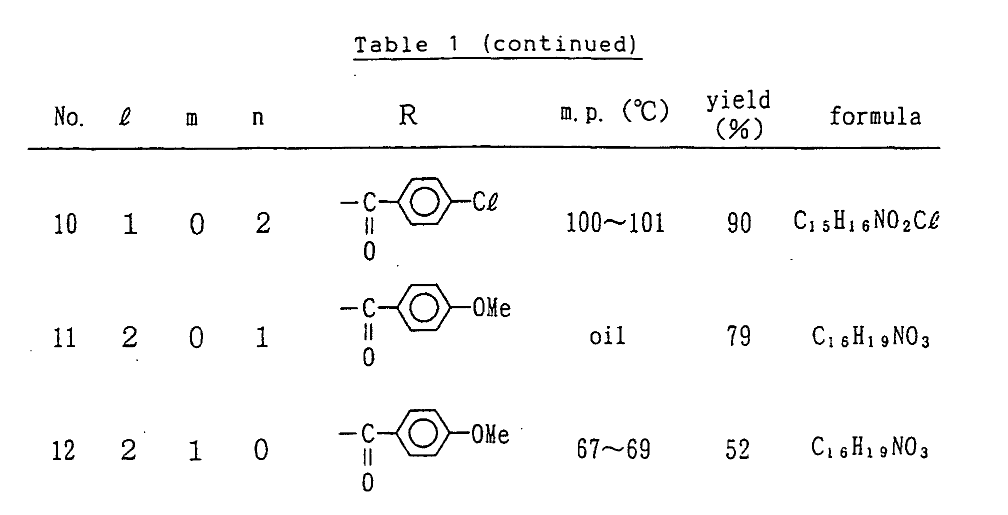

### Reference Example 6

### Preparation of 6-oxo-bicyclo[3.3.0]octan-2-one=ethylene=acetal (Compound B)

A 9.62 g quantity of bicyclo[3.3.0]octan-2,6-dione (Compound A), 0.265 g of p-toluenesulfonic acid monohydrate and 4.54 g of ethylene glycol were dissolved in 50 ml of benzene, and the mixture was refluxed for reaction for 6 hours while removing water as an azeotropic mixture. After the reaction, the mixture was cooled to room temperature and allowed to stand for 15 minutes with addition of 3 g of sodium hydrogencarbonate. The resulting precipitate was filtered off and washed with benzene. The filtrate was concentrated to obtain a brown oily product, which was purified by column chromatography using 180 g of silica gel and hexane-ethyl acetate (5:1) to obtain 9.28 g of Compound B mentioned above in the form of a colorless oily substance (yield: 73 %).
¹H-NMR (CDCl₃) δ ppm : 1.80∼ 2.45 (m, 10H), 3.95 (s, 4H)

### Reference Example 7

### Preparation of γ-1,t-2-hydroxybicyclo[3.3.0]octan-6-one=ethylene=acetal (Compound C)

A 9.23 g quantity of Compound B obtained in Reference Example 6 was dissolved in 70 ml of methanol, and 1.93 g of sodium boron hydride was added to the solution while cooling the solution in an ice-methanol bath. The mixuture was returned to room temperature 30 minutes later, followed by further reaction for 1 hour. The methanol was thereafter distilled off, 70 ml of water was added to the residue, and the mixture was subjected to extraction with 100 ml of dichloromethane and 50 ml of dichloromethane twice. The dichloromethane layer obtained was washed with 50 ml of saturated aqueous sodium chloride solution, then dried over anhydrous sodium sulfate and distilled for the removal of solvent, giving a colorless oily product. The product was purified by column chromatography using 150 g of silica gel and hexane-ethyl acetate (4:1 to 2:1) to obtain 6.63 g of Compound C mentioned above in the form of a colorless oily substance (yield: 71 %).
¹H-NMR (CDCl₃) δ ppm : 1.75∼ 2.00(m, 8H) , 2.10∼ 2.80(m, 2H), 3.90∼ 4.18 (m, 1H), 3.94 (s, 4H)

### Reference Example 8

### Preparation of γ-1,c-2-(4-nitrobenzoyloxy)bicyclo-[3.3.0]octan-6-one=ethylene=acetal (Compound D)

A 3.68 g quantity of Compound C obtained in Reference Example 7, 6.68 g of p-nitrobenzoic acid and 10.5 g of triphenylphosphine were dissolved in 70 ml of tetrahydrofuran, and a solution of 7.00 g of diethyl azodicarboxylate in 10 ml of tetrahydrofuran was added dropwise to the solution with ice cooling over a period of 10 minutes. The mixture was stirred with ice cooling for 1 hour, then returned to room temperature and further reacted for 16 hours. The solvent was distilled off from the reaction mixture, 50 ml of ether and 30 ml of hexane were added to the residue, and the resulting mixture was allowed to stand in a refrigerator for 1 day. The resulting precipitate (triphenylphosphine oxide) was filtered off and washed with hexane-ether (2:1). The filtrate obtained was concentrated to obtain a yellow oily product. The product was purified by column chromatography using 90 g of silica gel, hexane and hexane-ethyl acetate (10:1), giving 5.34 g of Compound D mentioned above in the form of a light yellow oily substance (yield: 80 %).
¹H-NMR (CDCl₃) δ ppm : 1.60∼ 2.20(m, 8H), 2.45∼ 2.90(m, 2H), 3.94(s, 4H), 5.10∼ 5.23(m, 1H), 8.24(s, 4H)

### Reference Example 9

### Preparation of γ-1,c-2-hydroxybicyclo[3.3.0]octan-6-one=ethylene=acetal (Compound E)

A 12.85 g quantity of Compound D obtained in Reference Example 8 was dissolved in 100 ml of methanol, 15 g of an anion exchange resin, Amberlite IR400 (OH-) (product of Organo Co., Ltd.), was added to the solution, and the mixture was refluxed for 8 hours. Since the starting material still remained, 10 g of the anion exchange resin was further added to the mixture, followed by refluxing for 16 hours. The reaction mixture was returned to room temperature, filtered under High Flow Supercell (product of Nacalai Tesque Co., Ltd.) and washed with methanol. Filtration gave a filtrate, which was concentrated and purified by column chromatography using 100 g of silica gel and hexane-ethyl acetate (3:1), whereby 6.52 g of Compound E above-mentioned was obtained as a colorless oily substance (yiled: 92 %).
¹H-NMR (CDCl₃) δ ppm : 1.20∼ 2.15(m, 8H), 2.30∼ 2.80(m, 2H), 3.80∼ 4.10(m, 1H), 3.91(s, 4H)

### Reference Example 10

### Preparation of γ-1,c-2-benzyloxybicyclo[3.3.0]-octan-6-one=ethylene=acetal (Compound F)

A 6.40 g quantity of Compound E obtained in Reference Example 9 was dissolved in 40 ml of N,N-dimethylformamide, 2.08 g of 60 % sodium hydride was added to the solution, and the mixture was stirred at room temperature. The reaction temperature rose to about 45 °C with evolution of hydrogen. One hour later, the reaction mixture was cooled with ice, followed by addition of 6.3 ml of benzyl bromide. The mixture was returned to room temperature 30 minutes thereafter and stirred for 24 hours, followed by further reaction on a bath of 70 °C for 4 hours. The mixture was returned to room temperature, and 80 ml of ice water was added thereto. The resulting mixture was subjected to extraction with 60 ml of ether three times. The ethereal layer was washed with 20 ml of water three times and with 20 ml of saturated aqueous solution of sodium chloride and thereafter dried over anhydrous sodium sulfate. Removal of the solvent from the layer gave an oily product, which was then purified by column chromatography using 75 g of silica gel, and hexane and hexane-ether (15:1), whereby 6.65 g of Compound F mentioned above was obtained as an oily substance (yield: 70 %).
¹H-NMR (CDCl₃) δ ppm : 1.60∼ 2.10(m, 8H) , 2.40∼ 2.80(m, 2H), 3.50∼ 3.80(m, 1H), 3.90(s, 4H), 4.92(s, 2H), 7.31(s, 5H)

### Reference Example 11

### Preparation of γ-1,c-2-benzyloxybicyclo[3.3.0]-octan-6-one (Compound G)

A 1.94 g quantity of Compound F obtained in Reference Example 10 was dissolved in 10 ml of tetrahydrofuran, and the solution was stirred for 6 hours with 3 ml of 2N hydrochloric acid added thereto. The tetrahydrofuran was distilled off from the resulting reaction mixture, followed by extraction with 20 ml of ether twice. The ethereal layer was washed with 10 ml of saturated aqueous solution of sodium hydrogencarbonate twice and then with 5 ml of saturated aqueous solution of sodium chloride, and dried over anhydrous sodium sulfate. Removal of the solvent from the layer give 1.60 g of Compound G mentioned above as an oily substance (yield: 98 %).
¹H-NMR (CDCl₃) δ ppm : 1.30∼ 2.40(m, 8H) , 2.60∼ 3.00(m, 2H), 3.70∼ 3.90(m, 1H), 4.51(s, 2H), 7.33(s, 5H)

### Reference Example 12

### Preparation of γ-6,c-7-benzyloxy-2-(4-methoxybenzoyl)-2-azabicyclo[4.3.0]nonan-3-one (Compound Ja) and γ-6,c-7-benzyloxy-3-(4-methoxybenzoyl)-3-azabicyclo-[4.3.0]nonan-2-one (Compound Jb)

A 1.54 g quantity of Compound G obtained in Reference Example 11 was dissolved in 15 ml of tetrahydrofuran, followed by addition of 8 ml of water and thereafter by addition of 0.94 g of hydroxylamine 5 hydrochloride and 1.84 g of sodium acetate trihydrate. Tetrahydrofuran was subsequently added to obtain a homogeneous mixture, which was then stirred at room temperature for 5 hours. The tetrahydrofuran was distilled off from the reaction mixture, followed by extraction with 80 ml of ethyl acetate. The ethyl acetate layer was washed with 10 ml of water, with 10 ml of saturated sodium hydrogencarbonate solution and subsequently with 10 ml of saturated aqueous solution of sodium chloride, and thereafter dried over anhydrous sodium hydrogen sulfate. Distillation of the layer for the removal of 5 the solvent gave 1.64 g of oxime of Compound G as a colorless oily substance.

A 1.60 g of the oxime obtained was dissolved in 16 ml of benzene, followed by addition of 3.11 g of p-toluenesulfonyl chloride and then by addition of 2.27 ml of triethylamine with ice cooling. The mixture was stirred for 4 hours with ice cooling and subsequently for 2 hours at room temperature, and thereafter diluted with 50 ml of ether. The resulting solution was washed with 10 ml of water, with 10 ml of 2N hydrochloric acid twice and subsequently with 10 ml of 5 saturated aqueous solution of sodium chloride, and thereafter dried over anhydrous sodium sulfate. Removal of the solvent from the solution gave a yellow oily product. The product was dissolved in 50 ml of anhydrous benzene, followed by addition of 43 g of silica gel (Fuji Silysia, BW-300 as washed with 2N HCl and then thoroughly with water, and dried at 230 °C for 16 hours) and further by addition of anhydrous benzene in an amount of permitting stirring of the resulting mixture. The mixture was shaken on a water bath having a constant temperature of 25 °C for 18 hours. The reaction mixture was poured into a column packed with 20 g of silica gel, and 300 ml of benzene was passed through the column to cause an excess of p-toluenesulfonyl chloride to flow out. The solvent was thereafter changed for benzene-methanol (6:1) to obtain an eluate. The eluate still contained impurities and was therefore purified by column chromatography again using 30 g of silica gel, and chloroform and chloroform-methanol (50:1). The product was dried in a vacuum at room temperature to obtain 1.233 g of a light yellow oily substance. PMR analysis revealed that the product was a mixture of γ-6,c-7-benzyloxy-2-azabicyclo[4.3.0]nonan-3-one (Compound Ha) and γ-6,c-7-benzyloxy-3-azabicyclo[4.3.0]nonan-2-one (Compound Hb) approximately in the ratio of 2:1.

The mixture (1.18 g) obtained was dissolved in 15 ml of dichloromethane, 1.31 g of p-methoxybenzoyl chloride and 1.34 g of triethylamine were added to the solution, and the mixture was stirred at room temperature for 36 hours. With addition of 80 ml of ethyl acetate, the reaction mixture was washed with 20 ml of 2N hydrochloric acid twice, with 20 ml of saturated solution of sodium hydrogencarbonate twice and then with 10 ml of saturated aqueous solution of sodium chloride, and dried over anhydrous sodium sulfate. Removal of the solvent gave a brown oily product, which was then purified by column chromatography using 30 g of silica gel and hexane-ethyl acetate (4:1 to 3:1). The component eluted first was Compound Jb mentioned above and obtained in an amount of 0.576 g as a yellow oily substance (yield: 25 %). The component eluted thereafter was Compound Ja mentioned above. The fraction was distilled for the removal of the solvent and recrystallized from ethanol, giving 1.05 g of the compound (yield: 46 %).
Chracteristic Values of Compound Ja
m.p. (in ethanol solvent) : 108.5∼ 109.5 °C
¹H-NMR (CDCl₃) δ ppm : 1.30∼ 2.70(m, 10H) , 3.70∼ 4.00 (m, 1H), 3.83(s, 3H), 4.54(s, 2H), 6.87(d, 2H), 7.34(s, 5H), 7.58(d, 2H)
Chracteristic Values of Compound Jb
¹H-NMR (CDCl₃) δ ppm : 1.30∼ 3.30(m, 10H) , 3.60∼ 4.10 (m, 1H), 3.83(s, 3H), 4.52(s, 2H), 6.86(d, 2H), 7.33(s, 5H), 7.54(d, 2H)

### Reference Example 13

### γ-6,c-7-Hydroxy-2-(4-methoxybenzoyl)-2-azabicyclo-[4.3.0]nonan-3-one (Compound 13)

A 1.05 g quantity of Compound Ja obtained in Reference Example 12 was dissolved in 15 ml of dioxane, 0.50 g of 10 % palladium carbon (product of Wako Junyako Co., Ltd.) was added to the solution, and the air in the reactor was removed by an aspirator and replaced by hydrogen repeatedly twice. The mixture was thereafter stirred in the hydrogen atmosphere (1 atm.) for 16 hours. The catalyst was filtered off and washed with dioxane. The resulting filtrate was concentrated to obtain a colorless oily product, which was then purified by column chromatography using 15 g of silica gel and chloroform. Removal of the solvent from the product afforded crystals, which were further recrystallized from ether, giving 0.64 g of Compound 13 mentioned above in the form of a colorless powder (yield: 81 %).
m.p. (in diethyl ether solvent): 120∼ 121 °C
¹H-NMR (CDCl₃) δ ppm : 1.20∼ 2.70(m, 10H) , 3.84(s, 3H), 3.90∼ 4.30(m, 1H), 4.20(q, 1H), 6.88(d, 2H), 7.59(d, 2H)

### Example 1 Tablet

| | |
|---|---|
| Compound 1 | 30 mg |
| Microcrystalline cellulose | 50 mg |
| Hydroxypropyl cellulose | 20 mg |
| Lactose | 47 mg |
| Talc | 2 mg |
| Magnesium stearate | 1 mg |

By the usual method, the above ingredients in the proportions given were made into tablets each weighing 150 mg.

### Example 2 Granule

| | |
|---|---|
| Compound 3 | 10 mg |
| Lactose | 400 mg |
| Corn starch | 370 mg |
| Hydroxypropylmethyl cellulose | 20 mg |

The above ingredients in the proportions given were made into a granular preparation by the usual method in an amount of 800 mg per wrapper.

### Example 3 Capsule

| | |
|---|---|
| Compound 11 | 55 mg |
| Lactose | 50 mg |
| Corn starch | 50 mg |
| Microcrystalline cellulose | 94 mg |
| Magnesium stearate | 1 mg |

By the usual method, the above ingredients in the proportions given were made into a granular preparation in an amount of 250 mg in each capsule.

### Example 4 Injection

| | |
|---|---|
| Compound 13 | 10 mg |
| Sodium chloride | 3.5 mg |
| Distilled water for injections, | suitable amount |

were made into an injection by the usual method.

### Example 5 Syrup

| | |
|---|---|
| Compound 3 | 50 mg |
| Purified sucrose | 60 g |
| Ethyl para-hydroxybenzoate | 5mg |
| Butyl para-hydroxybenzoate | 5mg |
| Perfume | suitable amount |
| Coloring agent | suitable amount |
| Purified water | suitable amount |

The above ingredients in the proportions given were made into a syrup by the usual method.

### Example 6 Suppositories

| | |
|---|---|
| Compound 11 | 50 mg |
| Witepsol W-35 | 1400 mg |

(Trademark, a mixture of mono-, di- and triglyceride of saturated fatty acids from lauric acid to stearic acid, Dynamite Nobel Co., Ltd.)

By the usual method, the above ingredients in the proportions given were made into suppositories.

### Test Example 1

### Anticonflict Test

### 1. Expermental animals

Wistar rats (males weighing 140 to 160 g) were used for experiment in groups of 11 to 14.

### 2. Test agents and administration method

The test compound, diazepam or buspirone was suspended in a 0.5 % sodium carboxymethyl cellulose solution, and the suspension was orally given to the animal in a volume of 5 ml/kg one hour before the start of experiment.

### 3. Experimental method and result

With reference to a method described in "Process in Anxiolytics and Antidepressants," edited by Showa Ueki and Tatsuo Furukawa, Ishiyaku Shuppansha, 56∼ 59 (1981), the agents were tested using experimental boxes having a grid floor and a metal drinking tube in the floor. No water was supplied to the rats for 48 hours before the experiment. Upon lapse of first 24 hours, each group of rats were placed into the experimental box, permitted access to water for 30 seconds and caused to recognize the metal drinking tube. Upon lapse of further 24 hours with access to water prevented, the rats were placed into the box again and permitted access to water on condition that an electric current was passed between the metal drinking tube and the grid to give an electroshock for every 20 times of water drinking behavior to measure the frequency of water drinking behavior for 3 minutes. Table 2 shows the result. Compound 1, 3, 11 and 13 exhibited an increase in the frequency of water drinking behavior at doses of 0.001 to 1.0 mg/kg, producing an anxiolytic effect, but diazepam and buspirone were found almost ineffective.

**Table 2**

| Anticonflict Test | | |
|---|---|---|
| Test compound | Dose (mg/kg) | Frequency of water drinking behavior (times/3 min) |
| Control | | 100 |
| Compound 1 | 0.01 | 138 |
| | 0.1 | 140 |
| | 1.0 | 122 |
| Compound 3 | 0.001 | 126 |
| | 0.01 | 162 |
| | 0.1 | 164 |
| | 1.0 | 166 |
| Compound 11 | 1.0 | 133 |
| Compound 13 | 0.001 | 130 |
| | 0.01 | 183 |
| | 0.1 | 177 |
| | 1.0 | 169 |
| Diazepam | 1.0 | 96 |
| Buspirone | 1.0 | 103 |

The frequency is expressed by a value relative to the frequency of the control which is taken as 100.

### Test Example 2

### Elevated plus maze Test

### 1. Exaperimental animals

Wistar rats (males weighing 170 to 220 g) were used for experiment in groups of 4 to 9.

### 2. Test agents and administration method

The test compound, diazepam or buspirone was suspended in a 0.5 % sodium carboxymethyl cellulose solution, and the suspension was orally given to the animal in a volume of 5 ml/kg one hour before the start of experiment.

### 3. Experimental method and result

The test was conducted using an experimental device, Model BTA-2, Behavior Analysis System (product of Muromachi Kikai Co., Ltd.) with reference to a method described in Psychopharmacology, 99, 48∼ 53(1989). The device had a maze comprising pairs of opposed open arms having no side walls and positioned at a level of about 50 cm above the floor, closed arms having side walls and a center square at an intersection. Each group of rats orally given the test drug were placed in the center square, and the behavior of the animals was recorded for 5 minutes using a behavior analyzer. The ratio of the number of entries into the open arm to the number of entries into all the arms was determined for evaluating the anxiolytic effect. Table 3 shows the result. Compound 3 achieved an increase in the number of entries into the open arm with lapse of time when taken at a dose of 0.001 mg/kg or greater, thus exhibiting an anxiolytic effect.

**Table 3**

| Elevated Plus Maze Test | | |
|---|---|---|
| Test compound | Dose | Ratio of entries into open arm |
| Control | | 100 |
| Compound 3 | 0.001 | 195 |
| | 0.01 | 219 |
| | 0.1 | 248 |
| Diazepam | 1.0 | 103 |
| | 10.0 | 144 |
| Buspirone | 1.0 | 98 |
| | 10.0 | 93 |

The ratio of entries into the open arm is expressed by a value relative to the ratio attained by the control which is taken as 100.

### Test Example 3

### Muscle Relaxant Effect (Traction Method)

### 1. Experimental animals and administration method

Compound 3, Compound 13, diazepam or buspirone was suspended in a 0.5 % sodium carboxymethyl cellulose solution, and the suspension was orally administered to 3 to 4 week old male ddY mice (in groups of 5) in a volume of 10 ml/kg one hour before the start of experiment.

### 2. Experimental method and result

With reference to a method described in Japan. J. Pharmacol., 49. 337∼ 349(1989), the foreleg of the mouse was hung on a horizontal wire, having a diameter of 1.2 mm and fixed at a level of 30 cm, three times consecutively. If the hind leg did not touch the wire within 10 seconds each time, the result was interpreted as positive. Thus, ED₅₀ was determined for evaluation. Consequently, Compounds 3 and 13 exhibited no muscle relaxant effect even when given at a dose of 300 mg/kg. Diazepam and buspirone were 2.2 mg/kg and 427.8 mg/kg, respectively, in ED₅₀.

### Test Example 4

### Sedative Effect (Spontaneous locomotor activity)

### 1. Experimental animals and administration method

Compound 3, Compound 13, diazepam or buspirone was suspended in a 0.5 % sodium carboxymethyl cellulose solution, and the suspension was orally administered to 3- to 4-week-old male ddY mice (in groups of 5) in a volume of 10 ml/kg one hour before the start of experiment.

### 2. Experimental method and result

The test was conducted with reference to a method described in "Evaluation of Medicinal Efficacies (1), Pharmacological Test Method (I), Basic Lectures on Development of Pharmaceuticals," 50∼ 54(1971). More specifically, the group of mice were given the test drug and thereafter measured the amount of spontaneous locomotor activity for 10 minutes per mouse using Animex MK-110 (product of Muromachi Kikai Co., Ltd.). When the amount of activity was up to 50 % of the control group, the result was interpreted as positive to determine ED₅₀ for evaluation. Consequently, Compounds 3 and 13 exhibited no sedative effect even at a dose of 300 mg/kg. Diazepam and buspirone were 1.7 mg/kg and 149.7 mg/kg, respectively, in the above value.

### Test Example 5

### Effect on central nervous system depressant

### a. Pentobarbital anesthetic method

### 1. Experimental method and administration method

Compound 3, Compound 13, diazepam or buspirone was suspended in a 0.5 % sodium carboxymethyl cellulose solution, and the suspension was orally administered to 3- to 4-week-old male ddY mice (in groups of 5 to 10) in a volume of 10 ml/kg one hour before the start of experiment.

### 2. Experimental method and result

The test was conducted with reference to a method described in "Evaluation of Medicinal Efficacies (1), Pharmacological Test Method (I)., Basic Lectures on Development of Pharmaceuticals," 144∼ 145(1971). More specifically, the group of mice were intraperitoneally given pentobarbital at a dose of 40 mg/kg and checked for sleeping time. When the sleeping time was in excess of twice that of the control group, the result was interpreted as positive to determine ED₅₀ for evaluation. Consequently, Compound 3 was at least 300 mg/kg, and Compound 13 was 224.5 mg/kg in this value. Diazepam and buspirone were 0.53 mg/kg and 91.7 mg/kg, respectively, in the value.

### b. Ethanol enhancing method

### 1. Experimental method and administration method

Compound 3, Compound 13, diazepam or buspirone was suspended in a 0.5 % sodium carboxymethyl cellulose solution, and the suspension was orally administered to 3- to 4-week-old male ddY mice (in groups of 6) in a volume of 10 ml/kg one hour before the start of experiment.

### 2. Experimental method and result

The test was conducted with reference to a method described in Japan. J. Pharmacol., 49, 337∼ 349(1989). More specifically, the group of mice were intraperitoneally given 25 % ethanol at a dose of 20 ml/kg and checked for the time interval between loss and recovery of the righting reflex. When the time measurement was in excess of twice the measurement of the control group, the result was interpreted as positive to determine ED₅₀ for evaluation. Consequently, Compounds 3 and 13 produced no ethanol enhancing effect even at a dose of 300 mg/kg. Diazepam and buspirone were 0.48 mg/kg and 120.1 mg/kg, respectively, in the value.

### Test Example 6

### Anticonvulsant Effect (Pentylenetetrazol-induced Convulsion Method)

### 1. Experimental method and administration method

Compound 3, Compound 13, diazepam or buspirone was suspended in a 0.5 % sodium carboxymethyl cellulose solution, and the suspension was orally administered to 3- to 4-week-old male ddY mice (in groups of 6) in a volume of 10 ml/kg one hour before the start of experiment.

### 2. Experimental method and result

The test was conducted with reference to a method described in "Evaluation of Medicinal Efficacies (1), Pharmacological Test Method (I), Basic Lectures on Development of Pharmaceuticals," 167∼ 172(1971). More specifically, pentylenetetrazol was subcutaneously administered to the mouse at a dose of 150 mg/kg, and when the mouse did not die due to onset of convulsion within 60 minutes, the result was interpreted as positive to determine ED₅₀ for evaluation. Consequently, Compounds 3 and 13 exhibited no anticonvulsant effect even at a dose of 300 mg/kg. Diazepam and buspirone were 0.35 mg/kg and at least 300 mg/kg, respectively, in the value.

### Test Example 7

### Acute Toxicity Test

Five-week-old male ddY mice were used in groups of 4 to 5. The mice were orally given the test compound as suspended in a 0.5 % sodium carboxymethyl cellulose solution and thereafter observed for 3 days to measure the number of deaths and determine LD₅₀. Table 4 shows the result.

**Table 4**

| Acute Toxicity Test | |
|---|---|
| Test compound | Acute Toxicity LD₅₀ (mg/kg) |
| Compound 1 | 2000< |
| Compound 3 | 2000< |
| Compound 11 | 2000< |
| COmpound 13 | 3000< |

### INDUSTRIAL APPLICABILITY

The anxiolytic agent comprising a bicyclolactam derivative represented by the formula (1) as its effective component has a high anxiolytic effect, is reduced in side effects such as sedative, muscle relaxant, hypnotic and anticonvulsant effects and is low in toxicity. Accordingly, the agent of the present invention is useful for treating or preventing chronic or acute anxiety disorders (or anxiety and fear neuroses), such as panic disorder accompanied or not accompanied by agoraphobia, social phobia or simple phobia, obsessive-compulsive disorder (neurosis), stress disorder resulting from injury and systemic anxiety disorder, and other anxiety disorders, and also for relieving healthy persons and the aged of anxiety.

Additionally, the present invention is useful for treating or preventing the anxiety attendant on withdrawal symptoms due to drug dependance and/or drug addiction. Thus, the present invention is useful for allaying withdrawal symptoms due to alcohol dependence, nicotine dependence, cocaine dependence and benzodiazepine dependence and withdrawal symptoms due to other drug dependence.

## Claims

1. The use of a bicyclolactam derivative represented by the following formula (1) 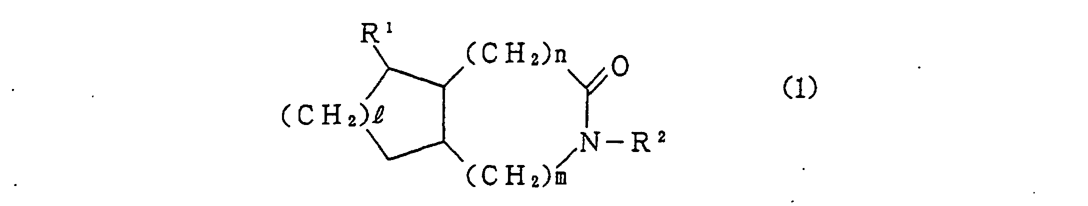 wherein R¹ is a hydrogen atom or hydroxyl group, R² is a benzoyl group which may optionally have at least one substituent, I is 1 or 2, m is 0 or 1 and n is 0, 1 or 2, provided that m and n do not represent 0 simultaneously, in the manufacture of a medicament for treating anxiety.

2. The use as defined in claim 1 wherein m is 0 in formula (1).

3. The use as defined in claim 1 wherein I is 1, m is 0 and n is 2 in formula (1).

4. The use as defined in claim 1 wherein I is 2, m is 0 and n is 1 in formula (1).

5. The use as defined in claim 1 wherein I is 2, m is 0 and n is 2 in formula (1).

6. The use as defined in any one of claims 1-5 wherein R² is a benzoyl group having as a substituent a halogen atom, lower C₁₋₆ alkyl group, lower C₁₋₆ alkoxyl group, nitro group, cyano group, hydroxyl group or amino group in formula (1).

7. The use as defined in claim 6 wherein R² is a benzoyl group having a lower C₁₋₆ alkoxyl group as a substituent in formula (1).

8. The use as defined in claim 7 wherein R² is a benzoyl group having a methoxy group as a substituent in formula (1).

9. The use as defined in claim 1 wherein R¹ is a hydroxyl group and I is 1, m is 0 and n is 2 in formula (1).

10. The use as defined in claim 1 wherein the compound is selected from:
2-(4-methoxybenzoyl)-2-azabicyclo[4.4.0]decane-3-one
2-(4-methoxybenzoyl)-2-azabicydo[4.3.0]nonane-3-one,
7-(4-methoxybenzoyl)-7-azabicyclo[4.3.0]nonane-8-one and
7-hydroxy-2-(4-methoxybenzoyl)-2-azabicyclo[4.3.0]nonane-3-one.

11. A bicyclolactam derivative represented by formula (1) according to claim 1, wherein R¹ is a hydroxyl group and R², l, m and n are as defined in claim 1.

12. A bicyclolactam derivative as defined in claim 11, wherein I is 1, m is 0 and n is 2 in formula (1).

13. 7-Hydroxy-2-(4-methoxybenzoyl)-2-azabicyclo[4.3.0]nonane-3-one.

14. A pharmaceutical composition comprising a bicyclolactam derivative as defined in any one of claims 11 to 13 and a conventional pharmaceutical carrier or excipient.

## Patentansprüche

1. Verwendung eines durch die folgende Formel (1) dargestellten Bicyclolactam-Derivats 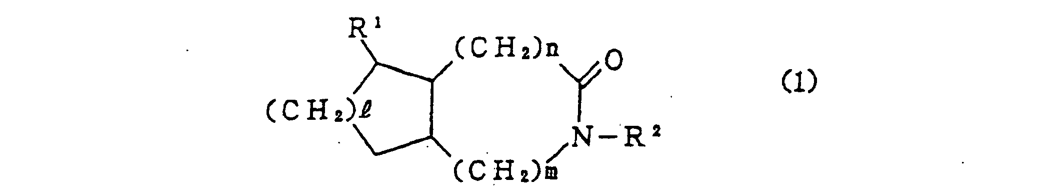 worin R¹ ein Wasserstoffatom oder eine Hydroxylgruppe ist, R² eine Benzoylgruppe ist, die gegebenenfalls mindestens einen Substituenten aufweisen kann, I 1 oder 2 ist, m 0 oder 1 ist und n 0, 1 oder 2 ist, mit der Maßgabe, dass m und n nicht gleichzeitig 0 darstellen, bei der Herstellung eines Arzneimittels zur Behandlung von Angst.

2. Verwendung wie in Anspruch 1 definiert, worin in Formel (1) m 0 ist.

3. Verwendung wie in Anspruch 1 definiert, worin in Formel (1) I 1 ist, m 0 ist und n 2 ist.

4. Verwendung wie in Anspruch 1 definiert, worin in Formel (1) I 2 ist, m 0 ist und n 1 ist.

5. Verwendung wie in Anspruch 1 definiert, worin in Formel (1) I 2 ist, m 0 ist und n 2 ist.

6. Verwendung wie in irgendeinem der Ansprüche 1 bis 5 definiert, worin in Formel (1) R² eine Benzoylgruppe ist, die als Substituenten ein Halogenatom, eine niedere C₁₋₆-Alkylgruppe, eine niedere C₁₋₆-Alkoxylgruppe, eine Nitrogruppe, eine Cyanogruppe, eine Hydroxylgruppe oder eine Aminogruppe aufweist.

7. Verwendung wie in Anspruch 6 definiert, worin in Formel (1) R² eine Benzoylgruppe ist, die eine niedere C₁₋₆-Alkoxylgruppe als Substituenten aufweist.

8. Verwendung wie in Anspruch 7 definiert, worin in Formel (1) R² eine Benzoylgruppe ist, die eine Methoxygruppe als einen Substituenten aufweist.

9. Verwendung wie in Anspruch 1 definiert, worin in Formel (1) R¹ eine Hydroxylgruppe ist und I 1 ist, m 0 ist und n 2 ist.

10. Verwendung wie in Anspruch 1 definiert, worin die Verbindung ausgewählt ist aus:
2-(4-Methoxybenzoyl)-2-azabicyclo[4.4.0]decan-3-on,
2-(4-Methoxybenzoyl)-2-azabicyclo[4.3.0]nonan-3-on,
7-(4-Methoxybenzoyl)-7-azabicyclo[4.3.0]nonan-8-on und
7-Hydroxy-2-(4-methoxybenzoyl)-2-azabicyclo[4.3.0]nonan-3-on.

11. Bicyclolactam-Derivat, dargestellt durch Formel (1) nach Anspruch 1, worin R¹ eine Hydroxylgruppe ist und R², l, m und n wie in Anspruch 1 definiert sind.

12. Bicyclolactam-Derivat wie in Anspruch 11 definiert, worin in Formel (1) I 1 ist, m 0 ist und n 2 ist.

13. 7-Hydroxy-2-(4-methoxybenzoyl)-2-azabicyclo[4.3.0]nonan-3-on.

14. Pharmazeutische Zusammensetzung umfassend ein Bicyclolactam-Derivat wie in irgendeinem der Ansprüche 11 bis 13 definiert und einen herkömmlichen pharmazeutischen Träger oder Exzipienten.

## Revendications

1. Utilisation d'un dérivé bicyclolactame représenté par la formule suivante : 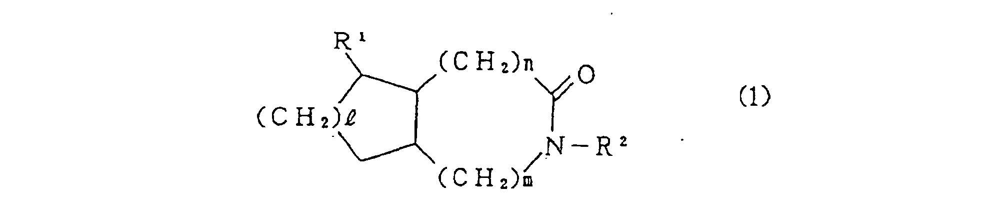 dans laquelle R¹ représente un atome d'hydrogène ou un groupe hydroxyle, R² représente un groupe benzoyle qui peut avoir éventuellement au moins un substituant, l est égal à 1 ou à 2, m est égal à 0 ou à 1 et n est égal à 0, à 1 ou à 2, à la condition que m et n ne représentent pas simultanément 0,
dans la fabrication d'un médicament pour le traitement de l'anxiété.

2. Utilisation selon la revendication 1, dans laquelle m est égal à 0 dans la formule (1).

3. Utilisation selon la revendication 1, dans laquelle l est égal à 1, m est égal à 0 et n est égal à 2 dans la formule (1).

4. Utilisation selon la revendication 1, dans laquelle l est égal à 2, m est égal à 0 et n est égal à 1 dans la formule (1).

5. Utilisation selon la revendication 1, dans laquelle l est égal à 2, m est égal à 0 et n est égal à 2 dans la formule (1).

6. Utilisation selon l'une quelconque des revendications 1 à 5, dans laquelle R² représente un groupe benzoyle ayant comme substituant un atome d'halogène, un groupe alkyle inférieur C₁₋₆, un groupe alcoxy inférieur C₁₋₆, un groupe nitro, un groupe cyano, un groupe hydroxyle ou un groupe amino dans la formule (1).

7. Utilisation selon la revendication 6, dans laquelle R² représente un groupe benzoyle ayant un groupe alcoxy inférieur C₁₋₆ comme substituant dans la formule (1).

8. Utilisation selon la revendication 7, dans laquelle R² représente un groupe benzoyle ayant un groupe méthoxy comme substituant dans la formule (1).

9. Utilisation selon la revendication 1, dans laquelle R¹ représente un groupe hydroxyle et l est égal à 1, m est égal à 0 et n est égal à 2 dans la formule (1).

10. Utilisation selon la revendication 1, dans laquelle le composé est choisi parmi :
- la 2-(4-méthoxybenzoyl)-2-azabicyclo[4.4.0]décane-3-one,
- la 2-(4-méthoxybenzoyl)-2-azabicyclo[4.3.0]nonane-3-one,
- la 7-(4-méthoxybenzoyl)-7-azabicyclo[4.3.0]nonane-8-one et
- la 7-hydroxy-2-(4-méthoxybenzoyl)-2-azabicyclo[4.3.0]nonane-3-one.

11. Dérivé bicyclolactame représenté par la formule (1) selon la revendication 1, dans laquelle R¹ représente un groupe hydroxyle et R², l, m et n sont tels que définis dans la revendication 1.

12. Dérivé bicyclolactame selon la revendication 11, dans lequel l est égal à 1, m est égal à 0 et n est égal à 2 dans la formule 1.

13. 7-Hydroxy-2-(4-méthoxybenzoyl)-2-azabicyclo[4.3.0]-nonane-3-one.

14. Composition pharmaceutique comprenant un dérivé bicyclolactame selon l'une quelconque des revendications 11 à 13, et un véhicule ou excipient pharmaceutique conventionnel.
